# EUROPEAN PATENT APPLICATION

(11) **EP 3 088 378 A1**
(43) Date of publication of application: **02.11.2016**
(21) Application number: 15305676.7
(22) Date of filing: 30.04.2015
(51) Int. Cl.: C07C 51/235, C07C 51/245

(54) **PROCESS FOR THE PREPARATION OF A MIXTURE OF ALDARIC ACIDS OR SALTS THEREOF**

(71) Applicant: Rhodia Operations, 75009 Paris (FR); Université Claude Bernard Lyon 1, 69100 Villeurbanne (FR); CENTRE NATIONAL DE LA RECHERCHE SCIENTIFIQUE, 75794 Paris Cedex 16 (FR)
(72) Inventor: MARION Philippe,, 69390 Lyon, (FR); DERRIEN, Elie,, 69003 Lyon (FR); PINEL Catherine,, 69007 Lyon, (FR); BESSON, Michèle,, 1700 Les Echets, (FR)
(74) Representative: Vande Gucht, Anne

(57) **Abstract**

The invention relates to a process for the preparation of a mixture of aldaric acids or salts thereof.

## Description

### Technical Field

The invention relates to a process for the preparation of a mixture of aldaric acids or salts thereof comprising reacting a mixture of reducing sugars with oxygen in the presence of an oxidation catalyst.

### Prior Art

Sugars are primary constituents of heterogeneous complex cellulosic biomass. They can be produced and recovered after selective hydrolysis of the biomass, sulfuric acid being known as a convenient reagent for this transformation. Intense research has been done on the highly efficient and selective conversion of glucose into high value-added chemicals. Gluconic and glucaric acids and their salts can be obtained by oxidation of aqueous glucose solutions using atmospheric oxygen or air in the presence of catalysts. Most of the investigations were oriented towards the selective oxidation of glucose to gluconic acid and gluconate, which is an important intermediate in food industry and pharmaceutical applications, and as a water-soluble cleaner for removing calcareous deposits (Besson, M. et al., Chem. Rev. 114, 1827 (2014)).

An increased interest was given to glucaric acid. This compound has been specified among the twelve most promising sugar-based building blocks for the chemical industry by the US Department of Energy. It is a promising raw material for biodegradable detergents, metal complexation agents and monomers. However, there are very few studies on the oxidation of glucose to glucaric acid, and the current synthesis methods are far from satisfactory.

US 2,427,168 describes the selective oxidation of glucose to glucarate in alkaline aqueous medium over 5 % Pt/C catalyst. US 2011/0306790 describes the selective oxidation of glucose to glucaric acid in aqueous solution over Au-Pt supported catalysts. US 2,472,168 and US 2011/0306790 employ pure D glucose as starting material.

Venema et al. (Journal of Molecular Catalysis, 77 (1992) 75-85) describe the aerobic oxidation of several pure aldopentoses in the presence of 5 % Pt/C. For lyxose and ribose the authors report a slower reaction rate arguing that a strong substrate-induced metal leaching is observed with these sugars.

WO 2010/144871 describes the selective oxidation of an aldopentose (xylose, ribose or arabinose) to pentaric acid over silica supported Pt cataysts.

However, in the prior art only oxidation of glucose to glucaric acid or one of some aldopentoses to pentaric acids is described. Therefore, there is a need for a process enabling the direct utilization of cellulosic material to produce a mixture of aldaric acids or salts thereof.

### Summary of the Invention

The authors of the present invention surprisingly found that a mixture of aldaric acids or salts thereof can be prepared by a process which comprises reacting a mixture of reducing sugars with oxygen in the presence of an oxidation catalyst. With the process according to the present invention it is possible to oxidize a mixture of reducing sugars comprising at least two reducing sugars that have a different number of carbon atoms.

Therefore, the invention relates to a process for the preparation of a mixture of aldaric acids or salts thereof, which process comprises reacting a mixture of reducing sugars with oxygen in the presence of an oxidation catalyst, wherein the mixture of reducing sugars comprises at least two reducing sugars that have a different number of carbon atoms.

The invention also relates to the use of an oxidation catalyst in the process for the preparation of a mixture of aldaric acids or salts thereof.

### Definitions

For purposes of the present description, certain terms are intended to have the following meanings.

The term "comprising" includes "consisting essentially of" and also "consisting of".

In addition, if the term "about" is used before a quantitative value, the present teachings also include the specific quantitative value itself, unless specifically stated otherwise. As used herein, the term "about" preferably refers to a ±10 % variation from the nominal value unless specifically stated otherwise.

### Brief Description of the Figures

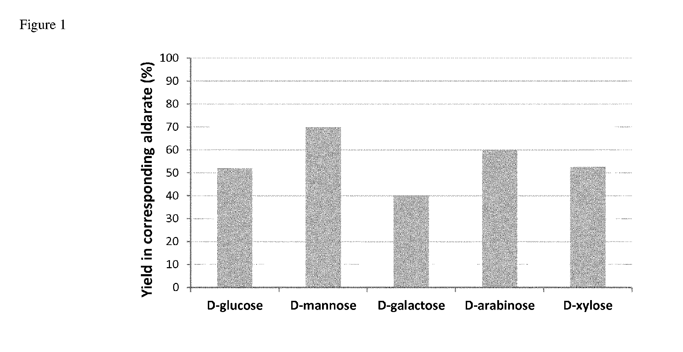

Figure 1 shows the results of example 2. The yields reported after 10 hour reaction are calculated on the basis of initial molar sugar concentration.

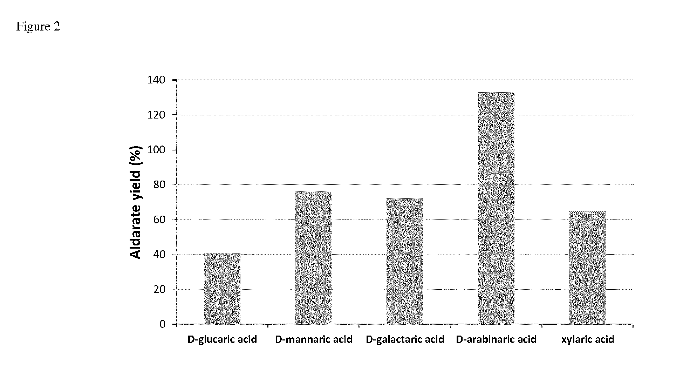

Figure 2 shows the results of the aerobic oxidation of mixed D-aldoses over Pt/C (example 3). The yields reported after 6 hours reaction are calculated on the basis of initial molar sugar concentration.

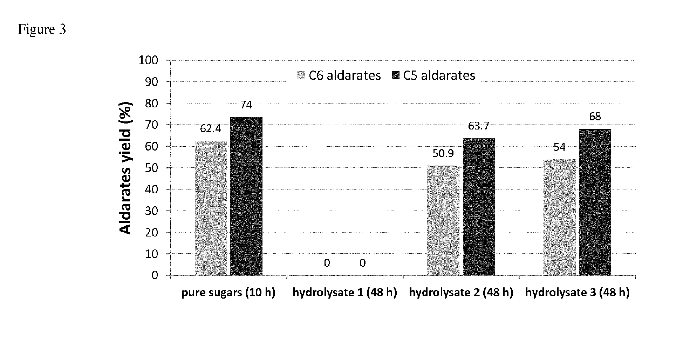

Figure 3 shows the results of the Pt catalyst aerobic oxidation of aldoses in hydrolysates (example 4). The yields are calculated on the basis of initial molar sugar concentrations.

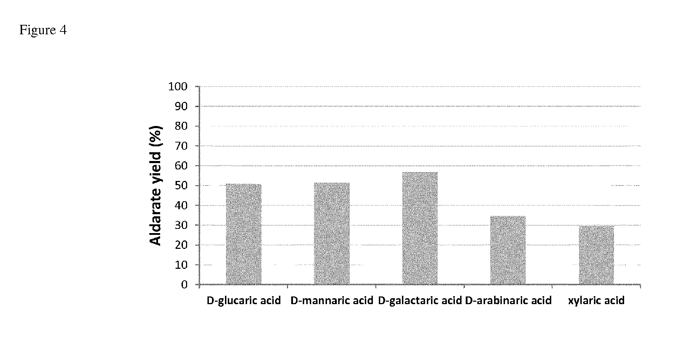

In Figure 4, the results of the aerobic oxidation of pure D-aldoses over Au-Pt/ZrO₂ are shown (example 6). The yields reported after 8 hours reaction are calculated on the basis of initial molar sugar concentration.

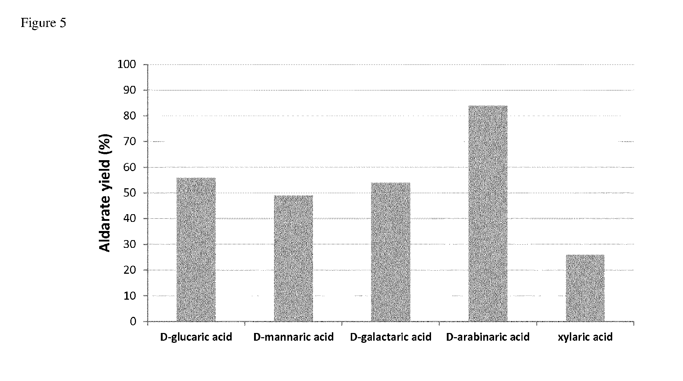

In Figure 5, the results of the aerobic oxidation of mixed D-aldoses over Au-Pt/ZrO₂ are shown (example 7). The yields reported after 8 hours reaction are calculated on the basis of initial molar sugar concentrations.

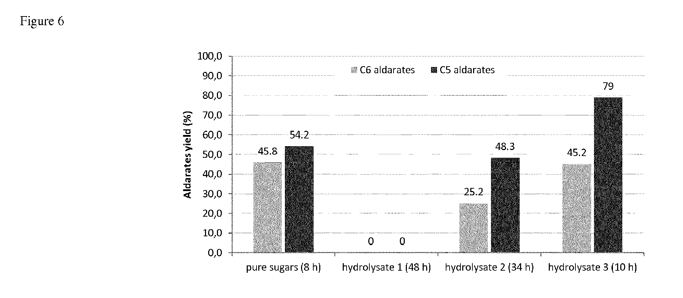

In Figure 6, the results of the Au-Pt catalyzed aerobic oxidation of aldoses in hydrolysates (example 8) are shown. The yields are calculated on the basis of initial molar sugar concentrations.

### Detailed Description of the Invention

The inventors of the present invention have surprisingly observed that a mixture of aldaric acids or salts thereof can be produced by reacting a mixture of reducing sugars with oxygen in the presence of an oxidation catalyst.

Therefore, the present invention relates to a process for the preparation of a mixture of aldaric acids or salts thereof, which process comprises reacting a mixture of reducing sugars with oxygen in the presence of an oxidation catalyst, wherein the mixture of reducing sugars comprises at least two reducing sugars that have a different number of carbon atoms.

In the context of the present invention a reducing sugar is any sugar that either has an aldehyde group or is capable of forming one in solution through isomerism. Examples of reducing sugars include monosaccharides, disaccharides, oligosaccharides and polysaccharides, as long as they have an open chain form with an aldehyde group or a free hemiacetal group. Monosaccharides which contain an aldehyde group are known as aldoses, and those with a ketone group are known as ketoses. Aldoses are grouped according to the number of carbon atoms and the carbon chain as dioses, trioses, tetroses, pentoses and hexoses. Glycoaldehyde is a diose and glyceraldehyde is a triose. Examples for tetroses include erythrose and triose, examples of pentoses include ribose, arabinose, xylose and lyxose and examples of hexoses include allose, altrose, glucose, mannose, gulose, idose, galactose and talose.

Also many disaccharides, like lactose and maltose have a reducing form as one of the two units may have an open chain form with an aldehyde group.

Preferably, the mixture of reducing sugars comprises aldopentoses and aldohexoses. Preferably, the aldopentoses according to the present invention include arabinose, lyxose, ribose and xylose. Most preferred are arabinose and xylose.

Preferably, the aldohexoses according to the present invention comprise allose, altrose, glucose, mannose, gulose, idose, galactose and talose. Most preferred are glucose, mannose and galactose.

As used herein, the reducing sugars refer to all possible stereoisomers of said sugar. Therefore, the "D-" and "L-" prefixes that are used for describing the stereoisomerism of monosaccharides are both encompassed if the term reducing sugar is used.

In one embodiment of the present invention, the mixture of sugars is in the form of a solution. In a preferred embodiment, said solution is an aqueous solution.

The term "solution" as used herein also includes a dispersion, as long as the reducing sugars can be reacted with oxygen in the presence of an oxidation catalyst.

In one embodiment of the present invention the mixture of reducing sugars is obtained by hydrolysis of cellulosic material. Cellulosic material as used herein includes renewable resources such as energy crops, plant biomass, agricultural wastes, forestry residues, sugar processing residues and plant-derived household wastes. More generally, renewable resources that may be used in accordance with the present invention include any renewable organic method that includes a source of hemicellulose such as, for example, switch grass, straw, oat halves, miscanthus, cassava, trees, vegetation, and crop residues. Other sources can include, for example, waste materials (e.g. spent paper, green waste, municipal waste, etc.).

The hydrolysis of cellulosic material is not limited to a specific method. A convenient reagent for the hydrolysis is sulfuric acid (R.J. Chimentao et al., Carbohydr. Polym. 2014, 111, 116-124).

The oxygen source in the process according to the present invention is not particularly limited. Preferably, the oxygen is supplied as air, oxygen enriched air, or oxygen, with one or more other constituents substantially inert to the reaction. Most preferably, the oxygen is supplied as air. The oxygen or oxygen-containing gas can be bubbled through the solution, for example under ambient pressure or at increased pressure.

In some embodiments, the air flow is in the range of about 5 to about 5,000 ml per minute, preferably about 100 to about 4,000 ml per minute, more preferably about 150 to about 2,000 ml per minute and most preferably in the range of about 200 to about 1,500 ml per minute, each in respect to about 100 to about 1000 ml of solution of reducing sugars.

The oxidation catalyst according to the present invention is not particularly limited. In some embodiments, the oxidation catalyst comprises at least one noble metal. In a preferred embodiment the noble metal is selected from the group consisting of Au, Pt and Pd.

In one embodiment, the oxidation catalyst is a supported catalyst. Preferably, the catalyst support is selected from the group consisting of carbon, ceria, titania and zirconia.

In another embodiment, the oxidation catalysts are Pt supported on active carbon, TiO₂, ZrO₂ or CeO₂ and other metal oxides.

In a preferred embodiment, the oxidation catalyst is Pt/C.

In another preferred embodiment, the oxidation catalyst comprises Au and Pt.

In an even more preferred embodiment, the oxidation catalyst is Au-Pt/ZrO₂ or Au-Pt/TiO₂.

The catalyst preparation methods include metallic sol immobilization, wet impregnation, incipient wetness impregnation and deposition-precipitation.

Deposition of Au and Pt can be successive or simultaneous.

The Pt:Au ratio may vary, for example, from about 100:1 to about 1:10, from about 50:1 to about 1:10, from about 10:1 to about 1:10 or more preferably from about 3:1 to about 1:4. More preferably still, the Pt:Au molar ratio may vary for example from about 3:1 to about 1:2. Even more preferably, the molar ratio of Pt:Au is in the range of about 2:1 to about 1:2, for example of about 1:1.

The total metal loading on the final catalyst (i.e., excluding any metal that is from the support) is generally less than about 10 wt.-% relative to the total catalyst weight. Preferably, the total metal loading ranges from about 1 to about 8 wt.-% relative to the total catalyst weight.

In one embodiment of the present invention the oxidation catalyst is Pt/C and during the reaction with oxygen the solution is maintained at a basic pH. In a preferred embodiment, the pH of this solution during the reaction with oxygen is maintained at equal to or above about 8, more preferably in the range of about 8 to about 10, and most preferably at about 9.

In another embodiment, the oxidation catalyst is Au-Pt/TiO₂ or Au-Pt/ZrO₂ and during the reaction with oxygen the pH is not regulated and decreases to a value of about 2 as the reaction proceeds.

In one embodiment, the oxidation catalyst is about 3.5 wt.-% Au and about 3.5 wt.-% Pt/ZrO₂.

Generally, the temperature during the reaction with oxygen is at least about 10°C, such as in the range of about 15°C to about 150°C, preferably in the range of about 25°C to about 125°C, more preferably in the range of about 25°C to about 100°C.

If the oxidation reaction is conducted in a solution, the temperature of the solution during the reaction with oxygen is at least about 10°C, preferably in the range of about 25°C to the boiling point of the solution. The temperature of the solution in the reaction with oxygen can for example be from about 50°C to the boiling point of the solution, preferably in the range of about 25°C to about 125°C, or about 25°C to about 100°C.

Typically, oxygen-containing gas pressure is in the range of about 1 to about 150 bar, preferably in the range of about 1 to about 80 bar, and most preferably about 40 bar.

In general, the oxidation reaction can be conducted in a batch, or semibatch reactor, or any other design that allows heterogeneous catalytic reactions. It should be understood that a reducing sugar, oxygen, any solvent, and the oxidation catalyst may be introduced into a suitable reactor separately or in various combinations. Particularly preferred is a pressurized Hastelloy reactor.

The initial concentration of reducing sugar can be in the range of about 1 to about 5,000 mmol/l, preferably about 10 to about 2,000 mmol/l, even more preferably in the range of about 50 to about 1,000 mmol/l and most preferably in the range of about 50 to about 500 mmol/l, such as about 250 mmol/l of the solution.

In a preferred embodiment, the parameters are set to an initial reducing sugar concentration of about 250 mmol/l of the solution, a temperature of about 100°C, and an oxygen-containing gas, preferably air pressure of about 40 bar.

In another embodiment, the parameters are set to an initial reducing sugar concentration of about 250 mmol/l of the solution, a temperature of about 60°C, a pH of about 9 and an oxygen-containing gas, preferably air flow of about 500 ml per minute.

In one embodiment, during the reaction with oxygen, the solution is stirred at about 100 to about 2,000 rpm, preferably at about 120 to about 1,200 rpm.

Furthermore, the present inventors found that if the solution containing the reducing sugars is obtained by hydrolysis of a cellulosic material, the reaction of the reducing sugars with oxygen can be adversely affected. In particular, the time required for the conversion of the reducing sugars to aldaric acids can be significantly increased. Upon further investigations, the inventors found that the solutions obtained by hydrolysis of cellulosic material contain impurities besides the desired reducing sugars. The inventors analyzed the solutions and found that these solutions generally contain sulfates, acetates, 5-hydroxymethylfurfural (HMF), furfural, maltose, cellobiose, raffinose, guaiacol and lignin besides the desired reducing sugars.

Upon further investigation, the inventors found that surprisingly only specific impurities adversely affect the reaction of the reducing sugar-containing solution with oxygen, namely HMF and furfural. The other impurities were found to have significantly less or even no influence on the reaction with oxygen.

Upon further investigations, the inventors found that nevertheless a certain residual amount of HMF and furfural is acceptable during the subsequent reaction with oxygen. This finding is particularly relevant on an industrial scale because removal of undesired impurities after the hydrolysis step is time consuming and costly. It is therefore of advantage, if the impurities can remain in the solution up to a concentration where they have no or only a little adverse influence on the reaction with oxygen.

Therefore, in a preferred embodiment, the solution of the reducing sugars used in the reaction with oxygen contains less than about 1.0 g/l of 5-hydroxymethylfurfural, preferably less than about 0.9 g/l, even more preferably less than about 0.8 g/l, or less than about 0.7 g/l and most preferably less than about 0.6 g/l of 5-hydroxymethylfurfural.

In a further preferred embodiment, the solution contains less than about 1.0 g/l of furfural. For example, the solution contains less than about 0.9 g/l of furfural, preferably less than about 0.8 g/l, or less than about 0.7 g/l, and most preferably less than about 0.6 g/l of furfural.

In particular, the solution should contain the above lower concentrations of HMF and furfural at the same time, whereby all upper limits of the concentration of HMF may be combined with all upper limits of the concentration of furfural.

In another preferred embodiment, the solution contains less than about 1.0 g/l of 5-hydroxymethylfurfural and less than about 1.0 g/l of furfural. Even more preferably, the solution contains less than about 0.9 g/l of 5-hydroxymethylfurfural and less than about 0.9 g/l of furfural. Even more preferably, the solution contains less than about 0.6 g/l of 5-hydroxymethylfurfural and less than about 0.6 g/l of furfural.

In order to reduce the amount of 5-hydroxymethylfurfural and/or furfural, the solution may be treated prior to the reaction with oxygen by at least one treatment step selected from ion exchange chromatography, active carbon treatment, and distillation (concentration). In a preferred embodiment, the solution is treated by ion exchange chromatography following active carbon treatment which may be followed by distillation. Other treatments for reducing the amount of HMF and/or furfural in a solution are known to the skilled person.

Another aspect of the present invention is the use of an oxidation catalyst in the above described process.

The following examples are intended only to exemplify the invention and are not intended to limit the scope of the claimed invention.

### Examples

### Analysis

Samples of reaction groups were analyzed using a Metrohm 850 ion chromatograph. The products were separated on a CarboPac^{®} PA1 column and then quantified by amperometric detection. External calibration with standards was used to determine glucose conversion and yields of alderates.

### Example 1 - Preparation of 4.5 wt.-% Pt/C catalyst

In a 500 ml round bottomed flask, 19.91 g of dry carbon (CECA L3S) is dispersed in 200 ml ultrapure water under nitrogen atmosphere. An aqueous solution of H₂PtCl₆ (H₂PtCl₆ • 6H₂O 99.99 % Sigma Aldrich) containing 1.30 g Pt is slowly added to the suspension under stirring which is maintained for 4 hours. This suspension is cooled to 0°C. 55 ml of 37 wt.-% formaldehyde and 30 ml of 30 wt.-% KOH are subsequently added dropwise. After 16 hours of stabilization, the solution is filtrated, washed with deionized water and dried under nitrogen flow at 60°C to obtain 4.5 wt.-% Pt/C.

### Example 2 - Oxidation of pure sugars

300 ml of a 0.25 M sugar solution in distilled water are loaded in a 1.0 1 double jacket glass reactor equipped with mechanical agitation, gas bubbling and controlled alkaline pH assured by automatic injection of 10 wt.-% NaOH. Nitrogen is bubbled through the solution during 15 minutes, then 2.07 g of the catalyst according to example 1 is added and the reactor is heated to 60°C. The pH regulation is then set to 9 and the reaction is started by switching from nitrogen to 500 ml per minute air and setting agitation to 1,200 rpm. Samples are filtrated through 0.45 µm PVDF membranes, diluted in ultrapure water and analyzed by ion chromatography.

The experiment was performed on 5 different sugars (D-glucose, D-galactose, D-mannose, D-xylose and D-arabinose), which results are shown in Table 1.

### Example 3 - Oxidation of a mixture of sugars

The procedure of example 2 is repeated on an equimolar mixture of D-glucose, D-galactose, D-mannose, D-xylose and D-arabinose with a total initial concentration of 0.25 M. The results of the experiments are shown in Table 2. The yield in D-arabinaric acid is particularly high because of the decarboxylation of C6 aldaric acids during reaction yielding C5 aldaric acids.

### Example 4 - Oxidation of sugars contained in pinewood hydrolysate

The procedure of the example 2 is applied to a pinewood hemicellulose hydrolysate and the results of the experiments are shown in Table 3. Hydrolysate 1 was treated by filtration and concentration. Hydrolysate 2 was treated by filtration, ion exchange chromatography and concentration. Hydrolysate 3 was treated by filtration, ion exchange chromatography, active carbon treatment and concentration.

### Example 5 - Preparation of 3.5 wt.-% Au - 3.5 wt.-% Pt/ZrO₂ catalyst

In a 1.0 1 glass reactor equipped with mechanical agitation, 7.53 g of dry ZrO₂ (Mel Chemicals ZXO) is dispersed in 500 ml ultrapure water under nitrogen atmosphere. An aqueous solution of H₂PtCl₆ (H₂PtCl6 • 6H₂O 99.99 % Sigma Aldrich) and HAuCl₄ (HAuCl₄ • 3H₂O 99.9+% Sigma Aldrich) containing 0.284 g Pt and 0.284 g Au is added to the suspension under stirring which is maintained for 1 hour (400 rpm). The suspension is then cooled to 0°C and a 100 ml aqueous solution containing 1.25 g NaBH₄ (NaBH₄ > 98 % Sigma Aldrich) is added dropwise under vigorous agitation (800 rpm). After 3 hours of stabilization, the solution is filtrated, washed with deionized water and dried under nitrogen flow at 60°C to obtain 3.5 wt.-% Au - 3.5 wt.-% Pt/ZrO₂.

### Example 6 - Oxidation of pure sugars

100 ml of a 0.25 M sugar solution in deionized water are loaded in a 300 ml Hastelloy reactor equipped with mechanical agitation, sampling tube, pressure and temperature regulation. Argon is bubbled through the solution during 15 minutes, then 0.883 g of the catalyst according to example 5 is added and the reactor is sealed. The reactor is flushed three times with 5 bar argon, then the temperature is raised to 100°C. The reaction is started by pressurizing the reactor to 40 bar air and setting agitation to 1,200 rpm. Samples are filtrated through 0.45 µm PVDF membranes, diluted in ultrapure water and analyzed by ion chromatography.

The experiment was performed on five different sugars (D-glucose, D-galactose, D-mannose, D-xylose and D-arabinose). The results of the experiment are shown in Table 4.

### Example 7 - Oxidation of a mixture of sugars

The procedure of the example 6 is repeated on an equimolar mixture of sugars (D-glucose, D-galactose, D-mannose, D-xylose and D-arabinose) totaling 0.25 M as initial concentration. The results of the experiments are shown in Table 5.

### Example 8 - Oxidation of sugars contained in pinewood hydrolysate

The procedure of example 6 is applied to a pinewood hemicellulose hydrolysate. The results of the experiments are shown in Table 6. Hydrolysate 1 was treated by filtration and concentration. Hydrolysate 2 was treated by filtration, ion exchange chromatography and concentration. 3 was treated by filtration, ion exchange chromatography, active carbon treatment and concentration.

**Table 1**

| Results of the aerobic oxidation of pure D-aldoses over Pt/C | | | | |
|---|---|---|---|---|
| Yields reported after 10 h reaction are calculated on the basis of initial molar sugar concentration. | | | | |
| Example | Substrate | Product | Conversion (%) | Yield (%) |
| 2 | D-glucose | D-glucaric acid | 100 | 52 |
| | D-mannose | D-mannaric acid | 100 | 70 |
| | D-galactose | D-galactaric acid | 100 | 40 |
| | D-arabinose | D-arabinaric acid | 100 | 60 |
| | D-xylose | xylaric acid | 100 | 53 |

**Table 2**

| Results of the aerobic oxidation of mixed D-aldoses over Pt/C | | | | |
|---|---|---|---|---|
| Yields reported after 6 h reaction are calculated on the basis of initial molar sugar concentration. | | | | |
| Example | Substrates | Products | Conversion (%) | Yield (%) |
| 3 | D-glucose | D-glucaric acid | 100 | 41 |
| | D-mannose | D-mannaric acid | 100 | 76 |
| | D-galactose | D-galactaric acid | 100 | 72 |
| | D-arabinose | D-arabinaric acid | 100 | 133 |
| | D-xylose | xylaric acid | 100 | 65 |

**Table 3**

| Results of the Pt-catalyzed aerobic oxidation of aldoses in hydrolysates. | | | | | | | | | | |
|---|---|---|---|---|---|---|---|---|---|---|
| Yields are calculated on the basis of initial molar sugars concentrations. | | | | | | | | | | |
| Example | Starting solution | Color | [hexoses]₀ (mM) | [pentoses]₀ (mM) | [HMF] (g/L) | [furfural] (g/L) | Conductivity (mS/cm) | Reaction time (h) | C6 yield (%) | C5 yield (%) |
| 4 | pure sugars | x | 181.5 | 70.3 | x | x | x | 10 | 62.4 | 74 |
| | hydrolysate 1 | yellow | 178.4 | 71.6 | 1.1 | 0 | 12 | 48 | 0 | 0 |
| | hydrolysate 2 | colorless | 161.7 | 47.4 | 0.5 | 0 | 0.00014 | 48 | 50.9 | 63.7 |
| | hydrolysate 3 | colorless | 110.6 | 45.3 | 0.0028 | 0 | 0.00034 | 48 | 54 | 68 |

**Table 4**

| Results of the aerobic oxidation of pure D-aldoses over Au-Pt/ZrO₂ | | | | |
|---|---|---|---|---|
| Yields reported after 8 h reaction are calculated on the basis of initial molar sugar concentration. | | | | |
| Example | Substrate | Product | Conversion (%) | Aldarate yield (%) |
| 6 | D-glucose | D-glucaric acid | 100 | 51 |
| | D-mannose | D-mannaric acid | 100 | 52 |
| | D-galactose | D-galactaric acid | 100 | 57 |
| | D-arabinose | D-arabinaric acid | 100 | 35 |
| | D-xylose | xylaric acid | 100 | 29 |

**Table 5**

| Results of the aerobic oxidation of mixed D-aldoses over Au-Pt/ZrO₂ | | | | |
|---|---|---|---|---|
| Yields reported after 8 h reaction are calculated on the basis of initial molar sugar concentration. | | | | |
| Example | Substrate | Product | Conversion (%) | Yield (%) |
| 7 | D-glucose | D-glucaric acid | 100 | 56 |
| | D-mannose | D-mannaric acid | 100 | 49 |
| | D-galactose | D-galactaric acid | 100 | 54 |
| | D-arabinose | D-arabinaric acid | 100 | 84 |
| | D-xylose | xylaric acid | 100 | 26 |

**Table 6**

| Results of the Au-Pt-catalyzed aerobic oxidation of aldoses in hydrolysates | | | | | | | | | | |
|---|---|---|---|---|---|---|---|---|---|---|
| Yields are calculated on the basis of initial molar sugars concentrations. | | | | | | | | | | |
| Example | Starting solution | Color | [hexoses]₀ (mM) | [pentoses]₀ (mM) | [HMF] (g/L) | [furfural] (g/L) | Conductivity (mS/cm) | Reaction time (h) | C6 yield (%) | C5 yield (%) |
| 8 | pure sugars | x | 136.0 | 108.7 | x | x | x | 8 | 45.8 | 54 |
| | hydrolysate 1 | yellow | 178.4 | 71.6 | 1.1 | 0 | 12 | 48 | 0 | 0 |
| | hydrolysate 2 | colorless | 161.7 | 47.4 | 0.5 | 0 | 0.00014 | 34 | 25.2 | 48.3 |
| | hydrolysate 3 | colorless | 110.6 | 45.3 | 0.0028 | 0 | 0.00034 | 10 | 45.2 | 79 |

## Claims

1. A process for the preparation of a mixture of aldaric acids or salts thereof, which process comprises reacting a mixture of reducing sugars with oxygen in the presence of an oxidation catalyst, wherein the mixture of reducing sugars comprises at least two reducing sugars that have a different number of carbon atoms.

2. The process according to any one of the preceding claims, wherein the mixture of reducing sugars comprises aldopentoses and aldohexoses.

3. The process according to claims 1 and 2, wherein the mixture of sugars is in the form of a solution, preferably an aqueous solution.

4. The process according to claim 3, wherein the mixture of reducing sugars is obtained by hydrolysis of cellulosic material.

5. The process according to any one of the preceding claims, wherein the oxygen is supplied as air, oxygen-enriched air, or oxygen, with one or more other constituents substantially inert to the reaction.

6. The process according to any one of the preceding claims, wherein the oxidation catalyst comprises at least one noble metal.

7. The process according to claim 6, wherein the at least one noble metal is selected from the group consisting of Au, Pt and Pd.

8. The process according to any one of the preceding claims, wherein the oxidation catalyst is a supported catalyst and the catalyst support is preferably selected from the group consisting of carbon, ceria, titania and zirconia.

9. The process according to any one of the preceding claims, wherein the oxidation catalyst is selected from the group consisting of Pt/C, Au-Pt/TiO₂ and Au-Pt/ZrO₂.

10. The process according to any one of claims 3 to 9, wherein the oxidation catalyst is Pt/C and during the reaction with oxygen the solution is maintained at a basic pH, preferably at a pH equal to or of above about 8.

11. The process according to any one of claims 3 to 9, wherein the oxidation catalyst is Au-Pt/TiO₂ or Au-Pt/ZrO₂ and during the reaction with oxygen the pH of the solution is not regulated.

12. The process according to any one of claims 3 to 11, wherein the solution contains less than 1.0 g/l of 5-hydroxymethylfurfural, preferably less than about 0.9 g/l of 5-hydroxymethylfurfural and most preferably less than about 0.6 g/l of 5-hydroxymethylfurfural.

13. The process according to any one of claims 3 to 12, wherein the solution contains less than about 1.0 g/l of furfural.

14. The process according to any one of claims 1 to 13, wherein the amount of 5-hydroxymethylfurfural and/or furfural is reduced prior to the reaction with oxygen preferably by at least one of the following treatment steps: (1) ion exchange chromatography, (2) active carbon treatment, and (3) distillation.

15. Use of an oxidation catalyst in the process according to any one of claims 1 to 14.
